(19) 

Europäisches
Patentamt
European
Patent Office
Office européen
des brevets

(11)  EP 3 722 788 A1

(12)  **EUROPEAN PATENT APPLICATION**
published in accordance with Art. 153(4) EPC

(43) Date of publication:
14.10.2020 Bulletin 2020/42

(21) Application number: 18887150.3

(22) Date of filing: 30.11.2018

(51) Int Cl.:
*G01N 21/51* $^{(2006.01)}$     *G01N 15/00* $^{(2006.01)}$

(86) International application number:
PCT/CN2018/118695

(87) International publication number:
WO 2019/109871 (13.06.2019 Gazette 2019/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 05.12.2017 CN 201711268447

(71) Applicant: Fatri United Testing & Control
(Quanzhou)
Technologies Co., Ltd.
Fujian 362000 (CN)

(72) Inventors:
• NIE, Yongzhong
Fujian 361008 (CN)
• ZHANG, Zhongping
Fujian 361008 (CN)

(74) Representative: Angerhausen, Christoph
Boehmert & Boehmert
Anwaltspartnerschaft mbB
Pettenkoferstrasse 22
80336 München (DE)

(54) **DEVICE AND METHOD FOR DETECTING FLUID TRANSPARENCY**

(57) The present invention discloses an apparatus for detecting fluid transparency and a detection method. The apparatus includes: a detection pipeline configured to allow a light beam to enter and exit from a fluid in the detection pipeline; a laser tube configured to output a light beam to enter the fluid; a photoelectric detector configured to detect a light beam exiting from the fluid; wherein, the photoelectric detector comprises a scatter detector and a transmission detector. The method includes: acquiring a scatter background noise value and a transmission background value, acquiring a scattered light intensity $I_{scatter}$ obtained by the scatter detector and a transmitted light intensity $I_{transmission}$ obtained by the scatter detector, and calculating an absorbed light intensity $I_{absorption}$ of a particle; acquiring a total light intensity $I_{total-light-intensity}$; obtaining the fluid transparency. According to the apparatus and the method, the accuracy of the detection of the fluid transparency can be effectively improved.

**FIG. 1**

EP 3 722 788 A1

**Description**

TECHNICAL FIELD

The present invention relates to the technical field of detection device, and in particular to an apparatus for detecting fluid transparency and a detection method performed using said apparatus.

BACKGROUND

In the operation process of an engine, a bearing, a gear and so on, a lubricating oil system is often required to be arranged so as to reduce abrasion loss, and factors such as insufficient purity of lubricating oil, existence of debris in lubricating oil will cause great disaster. A failed operation process of a large-scale mechanical equipment is often due to a vicious cycle caused by a continuous accumulation of abrasive debris in the lubricating oil. According to a diagnosis system for abrasion fault, there is a strong correlation between a damage degree of a worn component (for example, an engine, a rolling bearing, a gear, etc.) and particles in the lubricating system. In order to realize the detection of particles in the lubricating oil, a particle form detection apparatus, such as a fluid transparency detection apparatus, is usually arranged in a lubricating oil pipeline to monitor the quality of the lubricating oil on-line in real time, so as to provide an effective basis for fault diagnosis of an engine, a bearing, a gear, etc., and quickly and accurately determine the abrasion state and the reason of the fault.

In the existing methods for detecting liquid particles, usually, a laser light beam is guided to irradiate a transparent apparatus containing a liquid, and the particles in the liquid can be detected by measuring the light intensity of the scattered light. In this method, there are the following defects: (1) the scattered light is obtained in a single mode, so that the total light intensity of the light scattered in all directions cannot be accurately obtained, thus, the accuracy of the obtained total light intensity is not high; (2) the method for calculating the transparency of air is directly applied to the calculation of the transparency of the fluid without considering the influence of the inherent impurities of the fluid (that is, the background noise value of a pure fluid); both of these aspects will affect the accuracy of the calculation of the fluid transparency.

SUMMURY

In order to overcome the disadvantages of the prior art, the technical problems solved by the present invention are: (1) how to provide a detection apparatus capable of measuring the fluid transparency more accurately by detecting the scattered light intensity and the transmitted light intensity; (2) how to provide a detection method using said device to improve the accuracy of the detection of the transparency.

In order to solve the first technical problem, the technical solutions adopted in the present invention are as follows: An apparatus for detecting fluid transparency, including: a detection pipeline configured to allow a light beam to enter and exit from a fluid in the detection pipeline; a laser tube configured to output a light beam to enter the fluid; a photoelectric detector configured to detect a light beam exiting from the fluid; where, the photoelectric detector includes a scatter detector and a transmission detector.

In the prior art, in order to detect liquid particles, usually, a laser light beam is guided to irradiate a transparent apparatus containing a liquid, and the particles in the liquid can be detected by measuring the light intensity of the scattered light. Such technology will lead to the technical defects mentioned in the above background section. Therefore, the inventor has innovatively proposed the technical solution of arranged the transmission detector and the scatter detector in the apparatus at the same time, which can effectively solve the above technical problems, where the scattered light is obtained while the intensity of the transmitted light that is transmitted through the liquid can be obtained; and the total light intensity can be obtained by obtaining the transmitted light intensity, the scattered light intensity and the absorbed light intensity of the particle, so as to obtain the fluid transparency more accurately.

It should be noted that, the lubricating oil is taken as an example application scenario of the fluid in the present patent, which does not mean that the apparatus and the methods are only applicable to the application scenario of lubricating oil, and can also applied to other fluids.

It should be noted that, the scatter detector is mainly configured to obtain the scattered light information of the laser tube.

It should be noted that, the transmission detector is mainly used to obtain the transmitted light intensity of the laser tube.

Preferably, the scatter detector is arranged to be not on a same line with the light beam outputted by the laser tube.

The scatter detector is configured to identify the size and shape of particles. In the preferred embodiment, the scatter detector is arranged to be not on the same line with the light beam outputted by the laser tube. Because when the scatter detector is arranged to be on the same line with the light beam outputted by the laser tube, the sensitivity for the scatter detector to receive the light beam is low, and is likely to be interfered by direct light beams. When the scatter detector is not on the same line with the light beam outputted by the laser tube, the interference of the light beam can be reduced, and the detection of scattered light beam can be more accurate.

More preferably, the scatter detector is arranged on a vertical plane of the light beam outputted by the laser tube, and three position points of the scatter detector, the detection pipeline and the laser tube form a right-angled shape.

Experiments show that the interference of the direct light beams can be avoided to a greater extent through the arrangement of the above position relationship, and the accuracy of the detection of the scattered light beam can be ensured.

Preferably, the transmission detector is arranged to be on a same line with the light beam outputted by the laser tube, and the detection pipeline is arranged between the transmission detector and the laser tube.

Preferably, the apparatus further includes a drive device for driving movement of the laser tube.

In the existing apparatuses for detecting fluid transparency, the laser tube is fixed, and the outputted light beam is also fixed at a certain position, so that the particle detected is limited to the particle at that position. Since different sized particles are of different masses, the particles will be separated in different layers according to their masses. The existing apparatuses will lead to a narrow detection range, and the particles detected are relatively local to a certain position, resulting in the detected fluid transparency is not accurate enough. Therefore, the inventor has innovatively proposed to set laser tube (which is fixed according to the existing apparatuses) as movable, and the driving device is configured to drive the laser tube to move, so that the outputted light beam by the laser tube can cover the detection pipe, thus, the particles flowing through the entire detection pipeline are detected, which makes the light intensity obtained more accurate, so as to further improve the technical effects of improving the detection accuracy and precision.

Or, in another embodiment, the laser may be fixed to perform the measurement described in the above embodiment, and then in the next measurement process, the laser tube is moved to perform measurement in another layer. That is, in this preferred embodiment, the laser tube is not moved in a single measurement process, instead, the laser tube is fixed in a single measurement process, then the laser tube is moved after the single measurement process, and the laser tube is still fixed in another measurement process to measure the transparency. Finally, the result of each measurement process is recorded, and then the measurement result is obtained by weighted average algorithm, which makes the measurement result more accurate.

Preferably, the driving device is a motor.

In one the embodiment, a motor can be arranged in the apparatus to drive the laser tube to move, thus causing the outputted light beam that enters the fluid to move.

More preferably, the movement of the laser tube is screw rod movement or gear movement.

The screw rod movement refers to that, in one embodiment, a nut is arranged on the laser, and the motor driving screw cooperates with the nut to realize the movement.

The gear movement refers to that both the motor and the laser tube are provided with parts of gear like structures, and the parts cooperate with each other to realize the movement.

Preferably, a range of the movement of the laser tube is a diameter of a cross-section of the detection pipeline.

The range of the movement of the laser tube is set as the diameter of the cross-section of the detection pipeline, which can cover the detection pipeline to a greater extent, so that the fluid in the pipeline can be adequately covered by the light beam, therefore, the adequate detection of particle in each layer can be achieved and the detection accuracy can be improved.

Preferably, the movement of the laser tube is at a uniform speed.

In the process of uniform movement, the signal of the particle will be obtained more stably, which makes the result of the detection and analysis more accurate.

In order to solve the second technical problem, the technical solutions adopted in the present invention are as follows:

A method for detecting fluid transparency using the apparatus according to any one of claims 1 to 8, where, the method includes:

S1: introducing a pure fluid into the apparatus, obtaining a scatter background noise value and a transmission background noise value from the apparatus, and converting the scatter background noise value and the transmission background noise value into a corresponding scattered light intensity background noise value $I_{scattered\text{-}backgroung\text{-}noise}$ and a corresponding transmitted light intensity background noise value $I_{transmittion\text{-}background\text{-}noise}$;

S2: introducing a fluid under test into the apparatus, outputting a laser light beam by the laser tube, then obtaining a scattered light voltage signal and a transmitted light voltage signal obtained by the scatter detector and the transmission detector respectively, converting the scattered light voltage signal and the transmitted light voltage signal into a scattered light intensity $I_{scatter}$ obtained by the scatter detector and a transmitted light intensity $I_{transmission}$ obtained by the scatter detector, and obtaining an absorbed light intensity $I_{absorption}$ of a particle;

S3: outputting a total light intensity $I_{total\text{-}light\text{-}intensity}$ according to the results obtained in step S2, where, a $_{calculation}$

formula of the total light intensity is: $I_{total\text{-}light\text{-}intensity} = I_{scatter} + I_{transmission} + I_{absorption} - I_{scattered\text{-}backgroung\text{-}noise} - I_{transmittion\text{-}background\text{-}noise}$;

S4: outputting the fluid transparency T according to the $I_{total\text{-}light\text{-}intensity}$ and the $I_{transmission}$ obtained in S3, where a calculation formula of the fluid transparency T is:

$$T = (I_{transmission} - I_{transmittion\text{-}background\text{-}noise}) / I_{total\text{-}light\text{-}intensity} * 100\%.$$

It should be noted that, according to the existing methods for measuring fluid transparency, usually, the values of the total laser intensity and the transmitted light intensity are obtained directly, and then divide the two values directly to obtain the fluid transparency. However, in this manner, the total light intensity is hard to obtain and the accuracy of the directly obtained total light intensity is not high. Therefore, the inventor innovatively obtains the total light intensity step by step, that is, the scattered light intensity, the transmitted light intensity, and the absorbed light intensity of the particle are obtained respectively, and then the total light intensity is obtained by the addition of the scattered light intensity, the transmitted light intensity, and the absorbed light intensity of the particle. On the other hand, this manner is usually used for measurement in air, but there are many impurities which affect the scattering and transmission in the fluid compared with the air, so the manner for calculating the transparency of air cannot be directly applied to the calculation of the transparency of the fluid. This method can improve the accuracy of the detection of the total light intensity and the accuracy of the calculation of transparency.

Preferably, the laser tube is moved in S2;

More preferably, the laser tube is moved during the detection process;
More preferably, the laser tube is moved prior to the detection process.

The laser tube is movable, and there are two preferred solutions, both of the solutions can achieve the beneficial effect that the laser is not limited to entering the detection pipe at a certain point or in a certain layer, thus, the accuracy of each light intensity obtained by the detector will be improved.

The laser tube is moved during the detection process refers to that the laser moves during the process of obtaining the signals in S2, that is, particles in multiple layers can be detected that may be formed in the pipeline can be detected in real-time, and the forms of the particles in respective layers in the obtaining process, so that the accuracy of the signals can be improved.

In addition, the detection of the particle shape in the previous layer is performed in the process of laser detection of particles in the previous layer. In this way, higher signal accuracy can be obtained by weighted average of multiple detection results.

Preferably, after step S3, the method further includes: repeating step S2 and step S3 to obtain a maximum value of total light intensity $I_{max}$ within a period of time; and the fluid transparency $T = (I_{transmission} - I_{transmittion\text{-}background\text{-}noise}) / I_{max} * 100\%$.

It should be noted that, in the actual use of the lubricating oil, due to a series of problems such as the accuracy of data acquisition and the flow speed of the lubricating oil, the initially selected light intensity may not be the accurate total light intensity. In order to avoid the error in the calculation of the total light intensity, the total light intensity is calculated every time in the algorithm and compared with the previous calculated total light intensity. The maximum total light intensity in a period of time is selected as the actual total light intensity, which makes the measured total light intensity closer to the actual total light intensity and the result of the detection is more accurate.

Compared with the prior art, the invention has the following advantages:

1. In the apparatus for detecting fluid transparency according to the present invention, the scatter detector and the transmission detector are provided at the same time; by providing the two types of photoelectric detectors at the same time, two types of data can be detected in one apparatus at the same time, the transparency of the fluid in the detection pipeline can be further detected, and the detection efficiency is improved, that is, the scattered light is obtained while the intensity of the transmitted light that is transmitted through the liquid can be obtained; the accuracy of the detection of the transparency is improved;

2. In the apparatus for detecting fluid transparency according to the present invention, the laser tube is set to be movable, so that the light beam outputted by the laser tube can cover the detection pipeline, so as to detect the particles flowing through the whole detection pipeline, which further achieves the technical effect of improving the detection accuracy and precision;

3. In the apparatus for detecting fluid transparency according to the present invention, preferably, the motor is included, which is connected with the laser tube to drive the laser tube to move, and the mobility of the laser tube is realized;

4. In the apparatus for detecting fluid transparency according to the present invention, the range of the movement of the laser tube is set as the diameter of the cross-section of the detection pipeline, which can cover the detection pipeline to a greater extent, so that the fluid in the pipeline can be adequately covered by the light beam, therefore, the adequate detection of particle in each layer can be achieved and the detection accuracy can be improved.;

5. In the apparatus for detecting fluid transparency according to the present invention, the movement of the laser tube is at a uniform speed. In the process of uniform movement, the signal of the particle will be obtained more stably, which makes the result of the detection and analysis more accurate;

6. In the apparatus for detecting fluid transparency according to the present invention, the scatter background noise value and the transmission background noise value in the pure fluid are measured first to measure the inherent condition in the fluid, which is used as the basis for comparison in the subsequent measurement, so as to improve the accuracy of the subsequent measurement;

7. In the apparatus for detecting fluid transparency according to the present invention, the total light intensity is obtained step by step, that is, the scattered light intensity, the transmitted light intensity and the absorbed light intensity of the particle are obtained respectively, and then the total light intensity is obtained by adding the scattered light intensity, the transmitted light intensity and the absorbed light intensity of the particle and subtracting the background noise values. This method can improve the accuracy of the detection of the total light intensity, so that the accuracy of the calculation of the transparency is improved;

8. In the apparatus for detecting fluid transparency according to the present invention, step S2 and step S3 are repeated to obtain a maximum value of total light intensity $I_{max}$ within a period of time as the total light intensity, so that the measured total light intensity is more accurate.

The foregoing description is only an overview of the technical solutions of the present invention, and in order to make the technical means of the present invention more clearly understood and the present invention can be implemented in accordance with the content of the description, and in order to make the above and other objects, features, and advantages of the present invention more clearly understood, the following preferred embodiments are specifically described below with reference to the accompanying drawing.

BRIEF DESCRIPTION OF THE DRAWING

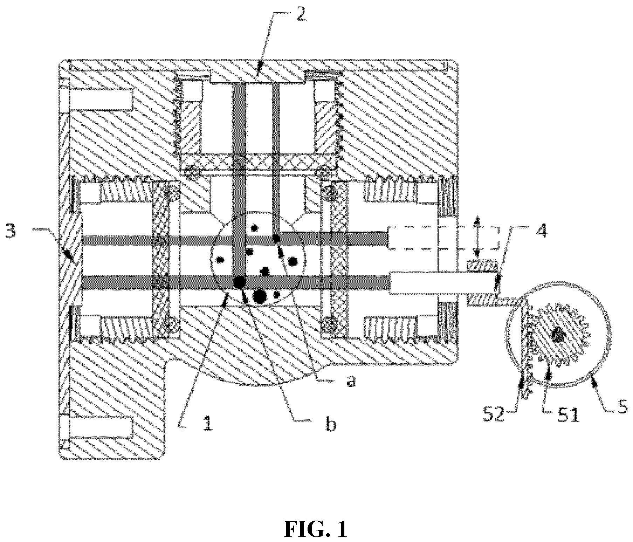

FIG. 1 is a structural diagram of a preferred implementation of an apparatus for detecting fluid transparency according to the present invention;
The reference sings include: 1: Detection pipe; 2: Scatter detector; 3: Transmission detector; 4: Laser tube; 5: Motor; 51: Gear; 52: Rack; a: Particle; b: Particle.

DESCRIPTION OF EXAMPLE CONFIGURATIONS

To further illustrate the technical means and effects of the present invention adopted to achieve the predetermined objects, embodiments, structures, characteristics and effects according to the present invention are described with reference to the accompanying drawing and preferred embodiments:

Embodiment 1 (an apparatus for detecting fluid transparency)

FIG. 1 illustrates a structural schematic diagram of a preferred embodiment of an apparatus for detecting fluid transparency according to the present invention, which includes: a detection pipe 1 configured to allow a light beam to enter and exit from a fluid in the detection pipeline; a laser tube 4 configured to output a light beam to enter the fluid; and a photoelectric detector configured to detect a light beam exiting from the fluid.

The photoelectric detector includes: a scatter detector 2 configured to detect a scattered light beam; a transmission detector 3 configured to detecting a transmitted light beam; the scatter detector is arranged on a vertical plane of the light beam outputted by the laser tube, and three points including positions of the scatter detector, the detection pipeline and the laser tube form a right-angled shape; and the transmission detector is arranged to be on the same line with the light beam outputted by the laser tube, and the detection pipeline is arranged between the transmission detector and the laser tube.

The detection device also includes a motor 5 which functions as a driving means, and the motor is connected with the laser tube to drive the movement of the laser tube. The motor drives the movement of the laser tube through the gear 51 and the rack 52.

The specific working process of the detection apparatus is as follows:
At the beginning, the motor, the gear and the rack are configured to control the laser tube to be positioned at the lowest position of the stroke to detect the liquid in the lowest layer of the pipeline, and the distribution of the particles in the lowest layer is analyzed; then a motor driving program is controlled to move the laser tube at a uniform speed, so that the laser light path moves in a scanning manner in the pipeline, signals outputted by the photoelectric detector are collected, and the distribution of particles in different layers of the pipeline is analyzed, thus realizing the function of dynamically analyzing the distribution of the particles in the pipeline. Since different sized particles (e.g., a relatively small particle "a" and a relatively large particle "b" in FIG. 1) are of different masses, they are separated in different layers, the light beam can be moved to enter the fluid in the detection pipeline in a covering manner using the apparatus, so that different particles are scattered and transmitted differently, the result of the detection performed by the photoelectric detector is more comprehensive and accurate, and the accuracy of the detection of the fluid transparency and the particle concentration is improved.

In the embodiment, the photoelectric detector includes the scatter detector and the transmission detector; by providing the two types of photoelectric detectors at the same time, two types of data can be detected in one apparatus at the same time, the transparency of the fluid in the detection pipeline can be further detected, and the detection efficiency is improved, that is, the scattered light is obtained while the intensity of the transmitted light that is transmitted through the liquid can be obtained; the accuracy of the detection of the transparency is improved.

In this embodiment, in order to reduce the interference of the light beam and make the detection of the scattered light beam more accurate, the scatter detector is arranged on a vertical plane of the light beam outputted by the laser tube, and three position points of the scatter detector, the detection pipeline and the laser tube form a right-angled shape. In other preferred embodiments, the scatter detector is arranged to be not on the same line with the light beam outputted by the laser tube, and the above technical effects can also be achieved.

In the embodiment, the laser tube is set to be movable, so that the light beam outputted by the laser tube can cover the detection pipeline, so as to detect the particles flowing through the whole detection pipeline, which further achieves the technical effect of improving the detection accuracy and precision.

In the embodiment, the movement of the laser tube is gear moving, and in other embodiments, the movement of the laser tube may also be screw rod moving, etc.

In the embodiment, a range of the movement of the laser tube is a diameter of a cross-section of the detection pipeline. The range of the movement of the laser tube is set as the diameter of the cross-section of the detection pipeline, which can cover the detection pipeline to a greater extent, so that the fluid in the pipeline can be adequately covered by the light beam, therefore, the adequate detection of particle in each layer can be achieved and the detection accuracy can be improved. In other embodiments, the moving range may be determined according to specific situations, and is not limited to the preferred implementation of this embodiment.

In the embodiment, the movement of the laser tube is at a uniform speed. In the process of uniform movement, the signal of the particle will be obtained more stably, which makes the result of the detection and analysis more accurate.

Embodiment 2 (a method for detecting fluid transparency using the apparatus as described above according to the present invention)

The embodiment is a first preferred embodiment of a method for detecting fluid transparency using the device described above. The method includes the following steps:

S1: introducing a pure fluid into the apparatus, obtaining a scatter background noise value and a transmission background noise value from the apparatus, and converting the scatter background noise value and the transmission background noise value into a corresponding scattered light intensity background noise value $I_{scattered-backgroung-noise}$ and a corresponding transmitted light intensity background noise value $I_{transmission-background-noise}$;

S2: introducing a fluid under test into the apparatus, outputting a laser light beam by the laser tube, then obtaining a scattered light voltage signal and a transmitted light voltage signal obtained by the scatter detector and the transmission detector respectively, converting the scattered light voltage signal and the transmitted light voltage signal into a scattered light intensity $I_{scatter}$ obtained by the scatter detector and a transmitted light intensity $I_{transmission}$ obtained by the scatter detector, and obtaining an absorbed light intensity $I_{absorption}$ of a particle;

S3: outputting a total light intensity $I_{total-light-intensity}$ according to the results obtained in step S2, where, a calculation formula of the total light intensity is: $I_{total-light-intensity} = I_{scatter} + I_{transmission} + I_{absorption} - I_{scattered-backgroung-noise} - I_{transmission-background-noise}$;

S4: outputting the fluid transparency T according to the $I_{total-light-intensity}$ and the $I_{transmission}$ obtained in S3, where a calculation formula of the fluid transparency T is:

$$T = (I_{transmission} - I_{transmission-background-noise}) / I_{total-light-intensity} * 100\%;$$

Specifically, in the process of analysis of lubricating oil transparency, the backgroung noise value in the pure fluid is first measured, so as to remove the inherent factor in the fluid that affects the measurement of the parameters. After that, the scatter detector and the transmission detector are used to obtain the scattered light intensity $I_{scatter}$ and the transmitted light intensity $I_{transmission}$ respectively. Based on the optical signal conditioning circuit, a weak light intensity can be converted into a voltage pulse signal; the processed scattered light signal includes information related to the number and size of the particle in the lubricating oil; the processed transmitted light signal includes the transmitted light intensity through the lubricating oil. According to the structure design and circuit conversion, the voltage signal $U_{transmissioin}$ transmitted through the lubricating oil can be received by the transmission detector, and the amplitude of the signal represents the intensity of the light. In optical analysis of granularity, a scattered light intensity voltage signal $U_{scatter}$ is obtained.

Through the above steps, the scattered light intensity voltage signal $U_{scatter}$ of the particle can be obtained. The size of the particle is calculated, and the analysis of absorption coefficient of the particle is performed, so that the light intensity voltage signal $U_{absorption}$ absorbed by the particle can be obtained. There is a certain proportion relationship between $U_{scatter}$ and $U_{absorption}$: $U_{absorption} = K * U_{scatter}$. K is related to the material and the absorption coefficient of the particle, and can be corrected by calibration in practice.

Then, the scattered light intensity $I_{scatter}$, the transmitted light intensity $I_{transmission}$ and the absorped light intensity $I_{absorption}$ of the particle are obtained according to the above voltage signals. Then, the total light intensity $I_{total\text{-}light\text{-}intensity}$ is obtained by adding the $I_{scatter}$, the $I_{transmission}$, and the $I_{absorption}$ and subtracting the inherent background noise values of the pure fluid.

The total light intensity is obtained step by step, that is, the scattered light intensity, the transmitted light intensity and the absorbed light intensity of the particle are obtained respectively, and then the total light intensity is obtained by adding the scattered light intensity, the transmitted light intensity and the absorbed light intensity of the particle and subtracting the background noise values. This method can improve the accuracy of the detection of the total light intensity, so that the accuracy of the calculation of the transparency is improved.

Embodiment 3 (a method for detecting fluid transparency)

The embodiment is a second preferred embodiment of a method for detecting fluid transparency. The difference between this embodiment and the above embodiment 2 is that the laser tube is movable in S2. The laser tube is movable, so that the laser is not limited to entering the detection pipe at a certain point or in a certain layer, thus, the accuracy of each light intensity obtained by the detector will be improved.

In addition, the movement of the laser tube has another further beneficial effect. In another preferred embodiment, the laser may be fixed to perform the measurement described in the above embodiment, and then in the next measurement process, the laser tube is moved to perform measurement in another layer. That is, in this preferred embodiment, the laser tube is not moved in a single measurement process, instead, the laser tube is fixed in a single measurement process, then the laser tube is moved after the single measurement process, and the laser tube is still fixed in another measurement process to measure the transparency. Finally, the result of each measurement process is recorded, and then the measurement result is obtained by weighted average algorithm, which makes the measurement result more accurate.

The specific steps are as follows:

(1) Initially, the laser light is controlled to be at the lowermost layer of stroke (i.e., the lowermost layer within a diameter of the cross section of the detection pipe), sampling is performed on the photoelectric detector, an analysis on the particle in the lubricating oil and the transparency of the lubricating oil is performed at the same time, and the analysis result $x_0$ regarding the current position is recorded.

(2) The laser tube is controlled to be moved upward at a uniform speed, sampling is performed on the photoelectric detector, an analysis on the particle in the lubricating oil and the transparency of the lubricating oil is performed at the same time, and the analysis result $x_i$ regarding the current position is recorded.

(3) When the laser tube is moved to the uppermost layer of the stroke (the uppermost layer within the diameter of the cross-section of the detection pipe), sampling is performed on the photoelectric detector, an analysis on the particle in the lubricating oil and the transparency of the lubricating oil is performed at the same time, and the analysis result $x_{\frac{max}{2}-1}$ regarding the current position is recorded.

(4) The laser tube is controlled to be moved downward at a uniform speed, sampling is performed on the photoelectric detector, an analysis on the particle in the lubricating oil and the transparency of the lubricating oil is performed at the same time, and the analysis result $x_{\frac{max}{2}+i}$ regarding the current position is recorded.

(5) Similarly, when the laser tube is moved to the initial position of the stroke, the analysis result $x_{max-1}$ is obtained, and a single layered analysis is completed.

(6) A secondary analysis is performed on the overall result of the layer-by-layer analysis, so that the particle distribution and the fluid transparency in the entire pipeline is obtained through the analysis. It is considered that the form of the fluid in the pipeline does not change much in a short period of time, and the movement of the laser tube is reciprocating motion at a relatively uniform speed, therefore, it is considered that the results obtained by reciprocatingly scanning layer-by-layer are consistent, and the particle distribution and the fluid transparency in each layer can be obtained by a calculation of the average value.

$$x_i = \frac{x_i + x_{max-i-1}}{2}$$

The other implementations included in this embodiment are the same as the above-mentioned Embodiment 2 and will not be repeated here.

Embodiment 4 (a method for detecting fluid transparency)

The embodiment is a third preferred embodiment of a method for detecting fluid transparency. The difference between this embodiment and the above Embodiment 2 is that after step S3, the method further includes: repeating step S2 and step S3 to obtain a maximum value of total light intensity $I_{max}$ within a period of time; and the fluid transparency $T = (I_{transmission} - I_{transmittion-background-noise}) / I_{max} * 100\%$;

In the actual use of the lubricating oil, due to a series of problems such as the accuracy of data acquisition and the flow speed of the lubricating oil, the initially selected light intensity may not be the accurate total light intensity. In order to avoid the error in the calculation of the total light intensity, the total light intensity is calculated every time in the algorithm and compared with the previous calculated total light intensity. The maximum total light intensity in a period of time is selected as the actual total light intensity, which makes the measured total light intensity closer to the actual total light intensity and the result of the detection is more accurate.

The selection of the total light intensity is an iterative process. In the algorithm, when the total light intensity is selected, the total light intensity is compared with the previously selected total light intensity. The selection of the total light intensity focuses the maximum value from the beginning to the end, therefore, for each measurement, For each measurement, the maximum total light intensity can be obtained by only performing one calculation of total light intensity and one comparison.

The other implementations included in this embodiment are the same as the above-mentioned Embodiment 2 and Embodiment 3 and will not be repeated here.

Embodiment 5 (an apparatus for detecting fluid transparency)

The basic structure of the embodiment is: an apparatus for detecting particle form, which includes: a detection pipeline configured to allow a light beam to enter and exit from a fluid in the detection pipeline; a laser tube configured to output a light beam to enter the fluid; a photoelectric detector configured to detect a light beam exiting from the fluid; the detection apparatus further includes a drive device for driving movement of the laser tube.

In addition to the above basic structure, the following preferred solutions may also include:
Preferably, the driving device is a motor.

Preferably, the movement of the laser tube is screw rod movement or gear movement.

Preferably, a range of the movement of the laser tube is a diameter of a cross-section of the detection pipeline.

Preferably, the movement of the laser tube is at a uniform speed.

Preferably, the photoelectric detector includes a scatter detector.

Preferably, the scatter detector is arranged to be not on the same line with the light beam outputted by the laser tube.

Preferably, the scatter detector is arranged on a vertical plane of the light beam outputted by the laser tube, and three points including positions of the scatter detector, the detection pipeline and the laser tube form a right-angled shape.

Preferably, the photoelectric detector also includes a transmission detector.

Preferably, the transmission detector is arranged to be on the same line with the light beam outputted by the laser tube, and the detection pipeline is arranged between the transmission detector and the laser tube.

The above embodiments are only the preferred embodiments of the present invention, and cannot be used to define the protection scope of the invention. Any non-substantive changes and replacements made by those skilled in the art on the basis of the present invention belong to the protection scope claimed by the present invention.

## Claims

1. An apparatus for detecting fluid transparency, comprising:

   a detection pipeline configured to allow a light beam to enter and exit from a fluid in the detection pipeline;
   a laser tube configured to output a light beam to enter the fluid;
   a photoelectric detector configured to detect a light beam exiting from the fluid;
   wherein, the photoelectric detector comprises a scatter detector and a transmission detector.

2. The apparatus according to claim 1, wherein the scatter detector is arranged to be not on a same line with the light beam outputted by the laser tube.

3. The apparatus according to claim 2, wherein, the scatter detector is arranged on a vertical plane of the light beam outputted by the laser tube, and three position points of the scatter detector, the detection pipeline and the laser tube form a right-angled shape.

4. The apparatus according to any one of claims 1 to 3, wherein, the transmission detector is arranged to be on a same line with the light beam outputted by the laser tube, and the detection pipeline is arranged between the transmission detector and the laser tube.

5. The apparatus according to any one of claims 1 to 4, wherein, the apparatus further comprises a drive device for driving movement of the laser tube.

6. The apparatus according to claim 5, wherein, the drive device is a motor; and
   preferably, the movement of the laser tube is screw rod movement or gear movement.

7. The apparatus according to claim 5 or 6, wherein, a range of the movement of the laser tube is a diameter of a cross-section of the detection pipeline.

8. The apparatus according to any of claims 4 to 7, wherein, the movement of the laser tube is at a uniform speed.

9. A method for detecting fluid transparency using the apparatus according to any one of claims 1 to 8, wherein, the method comprises:

   S1: introducing a pure fluid into the apparatus, obtaining a scatter background noise value and a transmission background noise value from the apparatus, and converting the scatter background noise value and the transmission background noise value into a corresponding scattered light intensity background noise value $I_{scattered-backgroung-noise}$ and a corresponding transmitted light intensity background noise value $I_{transmittion-background-noise}$;
   S2: introducing a fluid under test into the apparatus, outputting a laser light beam by the laser tube, then obtaining a scattered light voltage signal and a transmitted light voltage signal obtained by the scatter detector and the transmission detector respectively, converting the scattered light voltage signal and the transmitted light voltage signal into a scattered light intensity $I_{scatter}$ obtained by the scatter detector and a transmitted light intensity $I_{transmission}$ obtained by the scatter detector, and obtaining an absorbed light intensity $I_{absorption}$ of a particle;
   S3: outputting a total light intensity $I_{total-light-intensity}$ according to the results obtained in step S2, wherein, a calculation formula of the total light intensity is: $I_{total-light-intensity} = I_{scatter} + I_{transmission} + I_{absorption} - I_{scattered-backgroung-noise} - I_{transmittion-background-noise}$;
   S4: outputting the fluid transparency T according to the $I_{total-light-intensity}$ and the $I_{transmission}$ obtained in S3, wherein a calculation formula of the fluid transparency T is:

$$T = (I_{transmission} - I_{transmittion\text{-}background\text{-}noise}) / I_{total\text{-}light\text{-}intensity} * 100\%;$$

preferably, the laser tube is moved in S2;
more preferably, the laser tube is moved during the detection process;
more preferably, the laser tube is moved prior to the detection process.

10. The method according to claim 9 wherein, after step S3, the method further comprises: repeating step S2 and step S3 to obtain a maximum value of total light intensity $I_{max}$ within a period of time; and the fluid transparency $T = (I_{transmission} - I_{transmittion\text{-}background\text{-}noise}) / I_{max} * 100\%$.

EP 3 722 788 A1

**FIG. 1**

### INTERNATIONAL SEARCH REPORT

| International application No. |
| --- |
| **PCT/CN2018/118695** |

| **A.** | **CLASSIFICATION OF SUBJECT MATTER** |
| --- | --- |

G01N 21/51(2006.01)i; G01N 15/00(2006.01)i

According to International Patent Classification (IPC) or to both national classification and IPC

| **B.** | **FIELDS SEARCHED** |
| --- | --- |

Minimum documentation searched (classification system followed by classification symbols)

G01N21/-; G01N15/-

Documentation searched other than minimum documentation to the extent that such documents are included in the fields searched

Electronic data base consulted during the international search (name of data base and, where practicable, search terms used)

CNPAT, CNKI, WPI, EPODOC: 流体, 透明度, 激光, 管道, 散射, 透射, 移动, 电机, 光强, 底噪, fluid, transparen+, laser, detect +, scatter+, transmi+, motor, background, noise, intensity

| **C.** | **DOCUMENTS CONSIDERED TO BE RELEVANT** | |
| --- | --- | --- |

| Category* | Citation of document, with indication, where appropriate, of the relevant passages | Relevant to claim No. |
| --- | --- | --- |
| PX | CN 107831143 A (FATRI XIAMEN TECHNOLOGIES LTD.) 23 March 2018 (2018-03-23) claims 1-10 | 1-10 |
| PX | CN 207457010 U (FATRI XIAMEN TECHNOLOGIES LTD.) 05 June 2018 (2018-06-05) description, paragraphs [0004]-[0032], and figure 1 | 1-8 |
| X | CN 104020130 A (BEIJING GANDAYUAN TECHNOLOGY CO., LTD.) 03 September 2014 (2014-09-03) description, paragraphs [0034]-[0046], and figures 1-2 | 1-8 |
| X | CN 102128814 A (ANHUI INSTITUTE OF OPTICS AND FINE MECHANICS, CHINESE ACADEMY OF SCIENCES) 20 July 2011 (2011-07-20) description, paragraphs [0032]-[0057], and figure 1 | 1-8 |
| A | US 20110255076 A1 (SICK ENGINEERING G.M.B.H.) 20 October 2011 (2011-10-20) entire document | 1-10 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **31 January 2019** | **27 February 2019** |

| Name and mailing address of the ISA/CN | Authorized officer |
| --- | --- |
| **State Intellectual Property Office of the P. R. China** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2018/118695**

| Patent document cited in search report | | | Publication date (day/month/year) | Patent family member(s) | | | Publication date (day/month/year) |
|---|---|---|---|---|---|---|---|
| CN | 107831143 | A | 23 March 2018 | None | | | |
| CN | 207457010 | U | 05 June 2018 | None | | | |
| CN | 104020130 | A | 03 September 2014 | None | | | |
| CN | 102128814 | A | 20 July 2011 | CN | 102128814 | B | 05 September 2012 |
| US | 20110255076 | A1 | 20 October 2011 | EP | 2378270 | B1 | 24 June 2015 |
| | | | | EP | 2378270 | A1 | 19 October 2011 |

Form PCT/ISA/210 (patent family annex) (January 2015)